# EUROPEAN PATENT APPLICATION

(11) **EP 1 561 459 A1**
(43) Date of publication of application: **10.08.2005**
(21) Application number: 05001478.6
(22) Date of filing: 25.01.2005
(51) Int. Cl.: A61K 9/48, A61K 9/54

(54) **Pharmaceutical hard capsule containing inorganic substance**

(30) Priority: 30.01.2004 JP 2004022624; 02.03.2004 JP 2004057099
(71) Applicant: Daiichi Suntory Pharma Co., Ltd., Tokyo 102-8530 (JP)
(72) Inventor: Nomura, Masaaki, Ohra-gun, Gunma-ken, 370-0708 (JP); Yoshida, Takuro Suntory Tatebayashiryou, Tatebayashi-shi, Gunma-ken, 374-0057 (JP)
(74) Representative: Albrecht, Thomas, Dr.

(57) **Abstract**

The objective of the present invention is to improve the preservation stability of drugs filled within the hard capsule. Especially, the objective of the present invention is to improve the preservation stability of drugs filled within the hard gelatin capsule which base material is gelatin.

The present invention provides a capsule which improvement is that inorganic substance is comprised within the capsule. The present invention also provides the hard capsule wherein the inorganic substance comprised therein is any one or more than one selected from the group consisting hydrated aluminum silicate, synthetic aluminum silicate, light anhydrous silicic acid, hydrated silicon dioxide and magnesium aluminometasilicate.

## Description

### Technical Field

The present invention relates to a hard capsule stably comprising any drug filled within, and more specifically, a hard capsule comprising penem antibiotic as a drug.

### Background Art

Since granule such as granulated powder is difficult to take, granule is generally filled in hard capsule for administration. Hard capsule makes it easier to control the dosage by comprising the drug in dosage unit, and further have an advantage of making the intake of drug easier by concealing the unpleasant taste and smell. For the present, gelatin capsule using gelatin as a base material, comprises the major part of the market for capsule. However, it is known that gelatin capsule has a disadvantage of capsule being insoluble in cases the drugs containing aldehyde group or carbonyl group in the molecule are filled, or aldehyde-like substance is developed due to drug degradation, and as a result crosslinked structure in gelatin protein is formed [Pharm. Tech. Japan, Vol.14 No.3 p67-76 (1998)].

To overcome these shortcomings, improvements such as succinizing a gelatin, being the main substrate of the gelatin capsule [Japanese Patent Laid-Open Publication Nos. S61-186314 and H07-252138], and further, adding polyethylene glycol (macrogol) to the succinated gelatin [Japanese Patent Laid-Open Publication No. H06-72862] were made. Also the capsules using cellulose derivative and starch as a base material in place of gelatin are developed [Japanese Patent Gazette No. 2552937 and Japanese Patent Laid-Open Publication No. 2000-202003]. However, these capsules are expensive due to the small market size and high manufacture's costs thereof. Further, the gelatin capsule dissolves rapidly in digestive tract and dose not vary in quality. Because of the very heavy demand of gelatin capsule, it has been desired to develop the technique of insoluble gelatin capsule when an aldehyde-like substance is filled therein.

It has been proposed some technique to solve this problem. For example, the insolubilizing of hard gelatin capsule may be obtained by containing antioxidant such as sodium hydrogen sulfite to prevent the generation of peroxide which is precursor of aldehyde-like substance (The 32nd Annual Conference of Pharmaceutics, Research Round-Table, Lecture Summery, p46-49, 1995, and The 8th Symposium of the Society of Prescription of Solid Drug Product; Lecture Summery, p61-68, 1998). Further, Japanese Patent Laid-Open Publication No. H08-99869 discloses the method of preventing the gelatin capsule to be insoluble by blending free radical trapping agent to filler of capsule. Additionally, Japanese Patent Laid-Open Publication No. 2000-26282 discloses that aminobutyric acid prevents the insolubilizing of hard gelatin capsule. Nevertheless, these additions of substances are aiming to prevent the insolubilizing of the capsule itself, and not aiming to stabilize the filler of the capsule.

Penem compound, one of the drugs to be filled in the hard capsule, is a non-natural, β-lactam compound which is designed to integrate the structures of penicillin and cephalosporin [ANTIBIOTICS & CHEMOTHERAPY, Vol.13, No.10, p.74-80, 1997; Japanese Patent Laid-Open Publication Nos. S61-207387, S63-162694, S60-222486, and S54-119486].

Of these penem compounds, sodium (+)-(5R,6S)-6-[(R)-1-hydroxyethyl]-7-oxo-3-[(R)-2-tetrahydrofuryl]-4-thia-1-azabicyclo[3.2.0]hept-2-ene-2-carboxylate (hereinafter referred to as "faropenem") is often used as oral therapeutic medicine against the various kinds of infection, and because of novel structure of penem ring, it not only shows the antibacterial activity against methicillin-sensitive *Staphylococcus aureus* (MSSA), *Streptococcus pyogenes* and *Streptococcus pneumoniae*, but also shows strong antibiotic activities against gram-positive bacteria such as penicillin-resistant *Pneumococcus* (PRSP), oral cavity *Streptococcus* and *Enterococcus,* which could not be treated with conventional β-lactam antibiotics. Faropenem is also reported to show extensive antibiotic activity against gram-negative bacteria such as influenza bacillus and anaecrobe such as *Bacteroides,* and is prospective antibiotic substance [ANTIBIOTICS & CHEMOTHERAPY, Vol.13, No.10, p.74-80, 1997].

Despite all advantages, it is known that as in the case of β-lactam compound, absorbability at digestive tract is not good compared to fat-soluble compound, when aqueous penem compound including faropenem is administered orally [Textbook of Pharmaceutics, Revised 2nd Edition, Akinobu Otsuka, et al., Nankodo]. Considering the circumstance, prodrug compound which free carboxyl group of faropenem is esterified by (5-methyl-2-oxo-1,3-dioxolene-4-yl)methyl group and made fat-soluble, namely, (5-methyl-2-oxo-1,3-dioxolene-4-yl)methyl (5R,6S)-6-[(R)-1-hydroxyethyl]-7-oxo-3-[(R)-2-tetrahydrofuryl]-4-thia-1-azabicyclo[3.2.0]hept-2-ene-2-carboxylate (hereinafter referred to as "faropenem daloxate") is developed [Japanese Patent Laid-Open Publication Nos. H06-72875 and H06-128267].

Above-mentioned faropenem daloxate is characterized by possessing extensive antibacterial activity especially against anaecrobe, and less toxic. Also, oral absorbability of faropenem daloxate is as good as 70% and higher, and is absorbed at upper part of small intestine, ester bond is hydrolyzed in the living body and converted into faropenem, then show the antibiotic activity.

### Disclosure of Invention

The present invention relates to a pharmaceutical agent filled in a capsule, especially a hard capsule stably containing drug. More specifically, the present invention provides a capsule wherein a hard gelatin capsule is prevented from the insolubilization, and the degeneration of functional coating applied to drug filled in a capsule.

With antibiotics, antibacterial activity is more effectively exerted when the period antibiotics sustain its blood concentration higher than effective *in vivo* blood concentration is longer. Accordingly, it is more preferable to develop a long-acting agent, in case of oral administration drug, in which antibiotic is gradually absolved by digestive tract, and exert lasting effect. The same is said for previously mentioned faropenem daloxate. Since faropenem daloxate is mainly absorbed at upper part of small intestine and not absorbed at any other part of the digestive tract, the inventors of the present invention had studied for a development of faropenem daloxate in long-acting agent.

For this purpose, granule is selected since passage of drugs through digestive tract can be averaged and individual differences on oral absorption rate and blood drug kinetics can be minimized, at first. Then, granule is studied to be filled in hard capsule for its convenience of intake. On top of that, the inventors had studied a development of composition granule from granule which rapidly dissolve in stomach (hereinafter referred to as "immediate release granule") and enteric coated granule which coating dose not dissolve at pH of endogastric digestive fluid but dissolves at pH of digestive fluid in small intestine after reaching the upper part of small intestine, and then the drug disintegrate and its pharmaceutical agent is dissolved, to control passage dose at the upper part of small intestine and transit time after the intake of faropenem daloxate.

The inventors of the present invention, to develop composition granule, have first prepared an enteric coated granule by enteric coating the core granules comprising faropenem daloxate, and then it is put to preservation test. The result showed that the dissolution time of pharmaceutical agent of enteric coating granule is delayed after the preservation thereof. The cause is thought to be that disintegration of faropenem daloxate comprised in core granule during preservation, followed by faropenem daloxate originated decompose by contacting enteric coating resulting its degeneration.

Generally speaking, in the case where degeneration of the enteric coating might happen, seal coat (sub-coat layer) is applied between core granule and enteric coating layer [The 32nd Annual Conference of Pharmaceutics, Research Round-Table, Lecture Summery, p46-49, 1995]. Given this, core granule is seal coated by the conventional manner. Although, dissolution time of pharmaceutical agent is improved compared to the enteric coated granule without application of seal coating, only applying the seal coat dose not make faropenem daloxate stable enough [see Example 1 after-mentioned]. From the result, the cause of degeneration of the enteric coating is thought to be not only the direct contact of faropenem daloxate originated resolvent to the enteric coating, but also because of gaseous resolvent which can cross the seal coat issuing and degenerating the enteric coating.

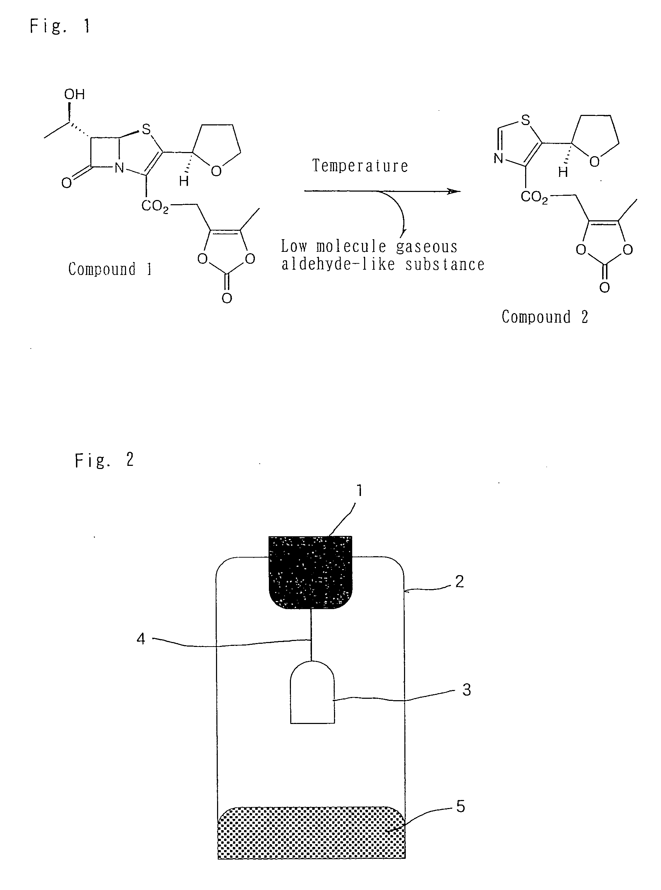

In addition, composition granule is prepared comprising the immediate release granule of which core granule of faropenem daloxate is applied with seal coat and the enteric granule of which the immediate release granule is applied with enteric coating, and subsequently filled into hard gelatin capsule and preserved. It is then observed that insolubilization of enteric coating is occurring [See Capsule-1/Tables 4 and 5 of Example 2 after-mentioned]. It is confirmed that 5-methyl-2-oxo-1,3-dioxolene-4-yl) 5-[tetrahydrofuryl]-thiazole-4-carboxylate (hereinafter referred to as "Compound 2") is generated due to primary degradation when faropenem daloxate is preserved under the severe temperature conditions [See Figure 1]. Compound 2 is generated as a result of cleavage of β-lactam ring of faropenem daloxate and low molecular, gaseous aldehyde-like substance is generated from the degradation mechanism at the same time with the generation of Compound 2. This gaseous aldehyde-like substance is believed to be the cause of insolubilization of hard gelatin capsule [See Figure 1].

Further as shown in Figure 2, faropenem daloxate is stored with hard gelatin capsule without any contact with the capsule in sealed glass container under 60° C (drifting moisture) for 7 days, and observed for the change. As shown in Table 1, generation quantity of Compound 2 has increased and hard gelatin capsule has insolubilized after 3 days of preservation. From the result, it is confirmed that insolubilization of hard gelatin capsule is caused by low molecular, gaseous aldehyde-like substance generated from degradation of faropenem daloxate.

By the way, faropenem daloxate is stated as "Compound 1" in Figures 1 and 2.

**Table 1:**

| Generation of Compound 2 and insolubilization of the capsule as in sealed preservation under the non-contact of faropenem daloxate and hard gelatin capsule | | | | | |
|---|---|---|---|---|---|
| Storage condition/Period | Initial | 60°C/drifting humidity/2 days | 60°C/drifting humidity/3 days | 60°C/drifting humidity/5 days | 60°C/drifting humidity/7 days |
| Generation quantity of Compound 2 (%) ¹⁾ | Not detected | Not detected | 0.02% | 0.04% | 0.07% |
| Dissolution of hard gelatin capsule ²⁾ | Completely dissolved | Completely dissolved | Thin, membranous fraction is left | Thin, membranous fraction is left | Membrane formed as capsule is left |

| | | | | | |
|---|---|---|---|---|---|
| ¹⁾: Generation quantity of Compound 2 is stated in ratio of peak area under Compound 2 to all peak area on the chromatogram from HPLC analysis. | | | | | |
| ²⁾: Body of the capsule after the preservation is soaked in 30mL of water in 37°C temperature, and visual observation is made 3 minutes later. | | | | | |

Consequently, adding anti-oxidation agent such as sodium hydrogen sulfate and free radical trapping agent as a prevention of free radical generation which is the source of the peroxide generation is examined as a method of preventing or inhibiting the generation of gaseous aldehyde-like substance. Nevertheless, there is a limit to amount used of these substances as a drug additive.

For example, maximum amount used as an oral preparation for sodium bisulfite is around 160mg, for sodium sulfite is 200mg, for tocopherol as a free radical trapping agent is 6mg, and for sodium pyrophosphate is 4mg [See Japan Drug Excipient Dictionary 2000, Japan Pharmaceutical Excipients Council, The Yakuji Nippo Limited]. As just described, in case amount used is very limited, preventing and inhibiting effect on generation of gaseous aldehyde-like substance is not adequate and there is a possibility where the insolubilization of the capsules can't be prevented.

In the case of faropenem daloxate, gaseous aldehyde-like substance is not generated through free radical as a source of peroxide but directly by degradation of faropenem daloxate, adding antioxidant and free radical dose not prevent nor inhibit the generation of gaseous aldehyde-like substance. Accordingly, the inventors of the present invention further studied the way to prevent the degeneration of the enteric coating due to the gaseous aldehyde-like substance generated by degradation of faropenem daloxate, and also to prevent the insolubilization of the hard gelatin capsule.

Thereat, the inventors of the present invention has attempted to fill an inorganic substance which is porous with its very large surface, very high oil absorbent and have high water absorption rates and confirmed safety on the human body as pharmaceutical preparation or drug adjuvant, with faropenem daloxate into the capsule. Consequentially, gaseous aldehyde-like substance generated by degradation of faropenem daloxate is absorbed by the inorganic substance which is filled in the capsule, and degeneration of enteric coating applied to drug believed to be caused by gaseous aldehyde-like substance is prevented. Further, insolubilization of the hard gelatin capsule is also confirmed to be prevented, and therefore, completing the invention.

The present invention provides a hard capsule stably containing any drugs filled within, and more specifically, a hard capsule in which insolubilization of the hard gelatin capsule and degeneration of the enteric coating applied to drug due to the gaseous substance generated by degradation of drugs are prevented by adding inorganic substance.

More specifically, the present invention provides:
(1) a hard capsule in which an inorganic substance is contained with a drug filled therein;
(2) a hard capsule according to (1), wherein base material of the hard capsule is gelatin;
(3) a hard capsule according to (1) or (2), wherein the inorganic substance contained therein is any one or more than one selected from the group consisting hydrated aluminum silicate, synthetic aluminum silicate, light anhydrous silicic acid, hydrated silicon dioxide and magnesium aluminometasilicate;
(4) a hard capsule according to (1) or (2), wherein the inorganic substance is magnesium aluminometasilicate;
(5) a hard capsule according to any one of (1) to (4), wherein the quantity of inorganic substance is within the range of 0.1 to 100 by weight% of the drugs filled within;
(6) a hard capsule according to any one of (1) to (5), wherein the drug filled in the hard capsule is in solid or semisolid form;
(7) a hard capsule according to (6), wherein solid and semisolid drug is fine granule or granule;
(8) a hard capsule according to any one of (1) to (7), wherein the drug filled within the hard capsule is applied with functional coating;
(9) a hard capsule according to (7), wherein said functional coating to be applied to the drug is an enteric coating;
(10) a hard capsule according to (7), wherein said functional coating to be applied to the drug is a gastric coating;
(11) a hard capsule according to any one of (1) to (10), wherein the drug filled within is a composition of a drug applied with functional coating and a drug without functional coating;
(12) a hard capsule according to (11), wherein the ratio of the composition of a drug applied with functional coating to a drug without functional coating is within the range of 3:7 to 7:3 in weight;
(13) a hard capsule according to any one of (1) to (7), wherein the drug filled within the hard capsule is a composition of a drug applied with enteric coating and a drug applied with gastric coating;
(14) a hard capsule according to (13), wherein the ratio of the composition of the drug applied with enteric coating to the drug applied with gastric coating is within the range of 3:7 to 7:3 in weight;
(15) a hard capsule according to any one of (1) to (14), wherein the drug filled in the hard capsule is penem antibiotic;
(16) a hard capsule according to (15), wherein penem antibiotic filled in the hard capsule is (+)-(5R,6S)-6-[(R)-1-hydroxyethyl]-7-oxo-3-[(R)-2-tetrahydrofuryl]-4-thia-1-aza-bicyclo[3.2.0]hept-2 -ene-2-carboxylic acid or pharmaceutically acceptable salt thereof and derivative thereof, or pharmacologically acceptable salt of derivative thereof;
(17) a hard capsule according to (15), wherein penem antibiotic filled in the hard capsule is (5-methyl-2-oxo-1,3-dioxolene-4-yl)methyl (5R,6S)-6-[(R)-1-hydroxyethyl]-7-oxo-3-[(R)-2-tetrahydrofuryl]-4-thia-1-azabicyclo[3.2.0]hept-2-ene-2-carboxylate;
(18) a hard capsule according to any one of (15) to (17), wherein the quantity of inorganic substance is within the range of 1 to 100 by weight% of the penem antibiotic filled within;
(19) a hard capsule according to any one of (15) to (17), wherein the quantity of inorganic substance is within the range of 1 to 25 by weight% of penem antibiotic filled within;
(20) a hard capsule according to any one of (15) to (19), wherein penem antibiotic filled within is a mixture of penem antibiotic applied with functional coating and penem antibiotic without functional coating;
(21) a hard capsule according to (20), wherein the ratio of a mixture of penem antibiotic applied with functional coating to penem antibiotic without functional coating is within the range of 5:5 to 7:3 in weight;
(22) a hard capsule according to any one of (15) to (19), wherein penem antibiotic filled within the hard capsule is a mixture of penem antibiotic applied with enteric coating and penem antibiotic applied with gastric coating;
(23) a hard capsule according to (22), wherein the ratio of a mixture of penem antibiotic applied with enteric coating to penem antibiotic applied with gastric coating is within the range of 5:5 to 7:3 in weight.

### Brief Description of Drawings

Figure 1 is the figure describing the chemical reaction of generation of low molecular gaseous aldehyde-like substance at the same time as generation of the compound 2 by degradation of faropenem daloxate.
Figure 2 is showing the testing condition of observation for change while faropenem daloxate is kept with hard gelatin capsule without any contact with the capsule in sealed glass container at 60° C (drifting moisture) for 7 days.
Figure 3 is the figure showing the transition of plasma concentration of faropenem when capsule-10 is orally administered to humans.
Figure 4 is the figure showing the transition of plasma concentration of faropenem when capsule-11 is orally administered to humans.
Figure 5 is the figure showing the transition of plasma concentration of faropenem when capsule-9 is orally administered to humans.
Figure 6 is the figure showing the transition of plasma concentration of faropenem when capsule-12 and capsule-13 are orally administered to humans.

In Figure 2, the signed numbers mean the following:
1: rubber stopper;
2: glass container
3: gelatin capsule
4: string
5: Compound 1

### Best Mode for Carrying Out the Invention

A penem antibiotic to be used for the present invention is not limited as long as it possesses antibacterial activity, sensitizing property and safety such as non oral toxicity, and is pharmaceutically acceptable. These can be free carboxylic acid, pharmaceutical salt such as salt with alkali metal or alkali-earth metal such as sodium, potassium, calcium, magnesium; amino acid salt such as lysine and ammonium salt. Of these, faropenem daloxate, a prodrug compound which free carboxyl group of faropenem is esterified by (5-methyl-2-oxo-1,3-dioxolene-4-yl)methyl group and made fat-soluble, is preferable.

The drug quantity to be contained within the capsule may be determined according to the kind of drug and disease to be treated. As in the case of comprising faropenem daloxate, 10 to 90 by weight% may be contained.

On the other hand, inorganic substance to be used for the present invention may mean the inorganic pharmaceutical adjuvant which is porous with its very large surface, have very high oil absorbent and water absorption rates, and possess good compression molding ability. Example of such inorganic substance is hydrated aluminum silicate, synthetic aluminum silicate, light anhydrous silicic acid, hydrated silicon dioxide and magnesium aluminometasilicate. The present invention is not limited by these examples, and may use one or more than two inorganic substance selected, if necessary.

The quantity of inorganic substance to be contained within the capsule may be determined according to the kind of inorganic substance selected and expected quantity of pharmaceutical degradation product, but within the range of 0.1 to 100 by weight% to the drug filled within the capsule is preferable, and even more preferable is the range of 1 to 25 by weight%.

These inorganic substances are widely in pharmacological use as fluidity improving agents for powdered medicine and granule, and also as diluents, binding agents, con-disintegration agents, anticaking agents and absorbent powder. However, to fill that within a capsule with medicinal properties for usage to absorb decomposition product of drugs is a novel matter of which the present inventors have found. In cases where these inorganic substances are used as fluidity improving agent, 0.1 to 1 by weight% to powdered medicine is generally added. The maximum oral administration quantity of the inorganic substance as a drug adjuvant is 504mg for kaolin (hydrated aluminum silicate), 1.8g for synthetic aluminum silicate, 2.6g for light anhydrous silicic acid, 3.8g for hydrated silicon dioxide and 1.05g for magnesium aluminometasilicate [See Japan Drug Excipient Dictionary 2000, Japan Pharmaceutical Excipients Council edit, The Yakuji Nippo Limited]. Accordingly, adjunction quantity may be selected within that range fitting the purpose of the present invention.

Of the capsule provided by the present invention, dosage form of the solid or semisolid drugs filled within the capsule may be powdered medicine, fine granule and granule. Such drug can be blended with drug as active ingredient, inorganic substance, and other additive, and may be produced in the conventional manner then filled. The inorganic substance may be contained within the film coating layer of powdered medicine, fine granule and granule. Such preparation can be filled within the capsule after simply mixing with inorganic substance, or each can filled separately.

For example, in the case of fine granule and granule applied with functional coating, excipient may be monosaccharide such as lactose, D-mannitol, sorbitol and glucose; polysaccharide such as dextrin, dextran and pullulan; starch such as corn starch, a-starch, carboxymethyl starch; and cellulose such as micro crystalline cellulose, micro crystalline cellulose-carmellose sodium.

Example of disintegrant is croscarmellose sodium, low substituted hydroxypropylcellulose and polyvinyl pyrrolidone. Binding agent, for example, can be hydroxypropylcellulose, hydroxypropyl methylcellulose, methylcellulose, hydroxyethylcellulose and polyvinyl pyrrolidone. Example of plasticizer is macrogol such as macrogol 400, macrogol 4000, macrogol 600 and macrogol 6000, polysorbate 80, triethyl citrate, sodium lauryl sulfate and sucrose esters of fatty acid. Aqueous film coating base material, for example, can be hydroxypropyl methylcellulose, hydroxypropylcellulose and carboxymethyl cellulose.

Additionally, functional film coating base material can be, for example, hydroxypropyl methylcellulose acetate succinate, hydroxypropyl methylcellulose phthalate, aminoalkyl methacrylate copolymer E (Eudragit® E100), aminoalkyl methacrylate copolymer RS (Eudragit® RS), methacrylic acid copolymer L (Eudragit® L100), methacrylic acid copolymer LD (Eudragit® L30D55), methacrylic acid copolymer S (Eudragit® S100) and ethyl acrylate methyl methacrylate copolymer dispersion (Eudragit® NE30D).

The adjunction quantity of the above mentioned additive may be determined considering the characteristic of the agent.

In the present invention, insolubilization of the hard gelatin capsule and degeneration of the enteric coating applied to drug filled in the capsule due to the gaseous substance generated by degradation of drug are prevented by adding inorganic substance to absorb gaseous aldehyde-like substance. It also makes the preparation design to carry out the desired function well as in long acting agent possible because degeneration of functional coating dose not occur, thus enabling to hold the target function.

The present invention is further described by the examples studying the aforementioned faropenem daloxate, but is not limited by them.

### Example 1:

According to Table 2 of composition ratio of components, enteric coated granule-1, enteric coated granule-2 and immediate release granule-1, in which those are containing faropenem daloxate, are prepared.

In other words, faropenem daloxate, lactose and corn starch are charged into agitating granulator and mixed. Separately, bider solution is prepared by dissolving hydroxypropylcellulose into water, and then added into agitating granulator for kneading and granulating. The resultant is then charged into basket-type granulator and granulated by extrusion method using 0.8mm screen. Subsequently, the resultant is charged into fluidized-bed granulator for fluidized-bed drying. The dried granules are then charged into sieving machine for granulating, put trough 18 mesh sieve, and to obtain remaining particle on the 30 mesh sieve as core granule.

The enteric coating solution 1 is prepared separately by dissolving or dispersing hydroxypropyl methylcellulose acetate succinate, triethyl citrate and talc into water. The resultant core granule is then charged into fluidized-bed granulator, and fluidized-bed coated with spraying enteric coating solution 1 to obtain desired enteric granule-1. The resultant core granule is charged into fluidized-bed granulator, and fluidized-bed coated with spraying seal coat solution prepared by dissolving or dispersing hydroxypropyl methylcellulose 2910 and talc into water to obtain desired immediate release granule-1. Also, hydroxypropyl methylcellulose acetate succinate, triethyl citrate and talc are dissolved or dispersed into water to prepare the enteric coating solution 2. The immediate release granule-1 is charged into fluidized-bed granulator, and fluidized-bed coated with spraying the enteric coating solution 2 to obtain desired enteric granule-2.

Both immediate release granule and enteric granule obtained are observed by electric microscope, and confirmed that coating is formed on granule. Hereinafter, each time the granule with the coating including seal coat is prepared, granule is observed by electric microscope and formation of coating film is confirmed before usage.

**Table 2:**

| Formula of the enteric granule-1, the enteric granule-2 and the immediate release granule-1. | | | |
|---|---|---|---|
| Components | Enteric granule-1 | Enteric granule-2 | Immediate release granule-1 |
| Faropenem daloxate(mg) (potency) | 642.6 (461.3mg) | 606.6 (435.5mg) | 849.1 (609.5mg) |

| Core granule components | | | |
|---|---|---|---|
| Lactose | 28.6 | 27.0 | 37.7 |
| Corn starch | 21.4 | 20.2 | 28.3 |
| Hydroxypropylcellulose | 21.4 | 20.2 | 28.3 |

| Seal coating components | | | |
|---|---|---|---|
| Hydroxypropyl methylcellulose 2910 | 0.0 | 15.0 | 21.2 |
| Talc | 0.0 | 25.0 | 35.4 |

| Enteric coating components | | | |
|---|---|---|---|
| Hydroxypropyl methylcellulose acetate succinate | 178.8 | 178.8 | 0.0 |
| Triethyl citrate | 53.6 | 53.6 | 0.0 |
| Talc | 53.6 | 53.6 | 0.0 |
| Total (mg) | 1,000.0 | 1,000.0 | 1,000.0 |

For the obtained enteric granule-1 and enteric granule-2, stability of these preparations is conducted under the storage condition described in Table 3. In another words, each of 1. 5g potency equivalent of granule-1 and enteric granule-2 preparations are filled hermetically in glass container and stored at 40°C under 75% relative humidity for one month. The faropenem daloxate content of these samples after the storage are then measured by HLPC method, and dissolution of faropenem daloxate form these preparations are also conducted by means of the dissolution test according to the Second Method (paddle method) of *Japanese Pharmacopoeia.*

The results are shown Table 3 below.

**Table 3:**

| Stability of enteric granule-1 and enteric granule-2 | | | | |
|---|---|---|---|---|
| Granule | Storage condition | Residual ratio (%) | Dissolution test (100mg potency equivalent of enteric granules are tested) | |
| | | | pH 6.5 solution | pH 6.8 solution |
| | | | 75% dissolution time of drug (min.) | 75% dissolution time of drug (min.) |
| Enteric granule-1 | Initial | 100 | 60 | 21 |
| | 40°C / 75% RH / 1 month | 97.5 | More than 60 | More than 60 |
| Enteric granule-2 | Initial | 100 | 60 | 21 |
| | 40°C / 75% RH / 1 month | 99.7 | More than 60 | 30 |

As the result shows, potency residual ratio of enteric granule-1 without seal coat after one month storage at 40°C under 75% relative humidity, is lowered to less than 98% and dissolution time of granules from the capsule is extended compared to the initial.

On the other hand, potency residual ratio of enteric granule-2 with seal coat after one month storage at 40°C under 75% relative humidity, is more than 98% and dissolution time of granules in pH6.8 solution are improved compared to enteric granule-1. Nevertheless, the dissolution time of granules in pH6.5 solution is not improved and thus, it is considered that only applying the seal coat dose not achieve sufficient stability effect.

### Example 2:

Each of the immediate release granule-1 and enteric granule-2 obtained in Example 1 are weighed for 40.0mg potency equivalent and 60.0mg potency equivalent quantity each, and filled in gelatin capsule to obtain capsule-1.

Aside from this, each of the immediate release granule-1 and enteric granule-2 obtained in Example 1 are weighed for 40.0mg potency equivalent and 60.0mg potency equivalent quantity each, and filled in gelatin capsule along with 20.0mg of magnesium aluminometasilicate as inorganic substance to obtain capsule-2.

**Table 4:**

| Formula of the gelatin capsules filled with immediate release granule-1 and enteric granule-2 | | |
|---|---|---|
| Components | Capsule-1 | Capsule-2 |
| | (Without magnesium aluminometasilicate) | (With magnesium aluminometasilicate) |
| Immediate release granule-1 | 65.6 (40.0mg potency) | 65.6 (40.0mg potency) |
| Enteric granule-2 | 137.8 (60.0mg potency) | 137.8 (60.0mg potency) |
| Magnesium aluminometasilicate | 0.0 | 20.0 |
| Fill ration per capsule (mg) | 203.4 | 223.4 |

The capsule-1 and the capsule-2 obtained are then assessed for stability under the storage condition shown in Table 5. That is, 12 capsules of each of capsule-1 and capsule-2 are sealed with 1g of silica gel in glass container and storages at 40°C under 75% relative humidity for four months, or at 25°C under drifting humidity for twelve months.

The faropenem daloxate content of these samples after the storage are then measured by HLPC method, and dissolution of faropenem daloxate form these capsules are also conducted by means of the dissolution test according to the Second Method (paddle method) of *Japanese Pharmacopoeia.*

The result is shown Table 5 below.

**Table 5:**

| Stability of gelatin capsules filled with immediate release granule-1 and enteric granule-2, in glass container for storage | | | | | | |
|---|---|---|---|---|---|---|
| Capsule | Storage condition | Residual ratio (%) | dissolution test | | | |
| | | | Water | pH5.5 solution | pH 6.0 pH 6.8 soln. soln. | |
| | | | Dissolution time of granule (min.) | 25% dissolution time of drug (min.) | 50% dissolution time of drug (min.) | |
| Capsule-1 | Initial | 100 | 2 | 15 | 24 | 18 |
| | 40°C/75% RH /4 months | 97.5 | 8 | Not assessed | More than 60 | More than 60 |
| | 25°C/drifting humidity/ 12 months | 97.1 | 12 | More than 60 | 28 | 24 |
| Capsule-2 | Initial | 100 | 2 | 15 | 24 | 18 |
| | 40°C/75% RH /4 months | 98.9 | 2 | Not assessed | 28 | 32 |
| | 25°C/drifting humidity/ 12 months | 98.2 | 2 | 17 | 22 | 20 |

As the result shows, the time in which granule is released from the capsule is extended and potency residual ratio is lowered to less than 98% and dissolution time of granule from the capsule is extended compared to the initial for capsule-1 without comprising magnesium aluminometasilicate, both after four months storage at 40°C under 75% relative humidity, and after 12 months storage at 25°C under drifting humidity.

On the other hand, the time in which granule is released from the capsule and dissolution time of granule from the capsule is same as the initial for capsule-2 which is comprising magnesium aluminometasilicate even stored in same condition as capsule-1. More over, potency residual ratio of capsule-2 is more than 98%, which is higher than that of capsule-1.

As shown above, magnesium aluminometasilicate indicated inhibition and prevention effect against the insolubilization of the gelatin capsule filled with granules containing faropenem daloxate, and decrease of dissolution rate and content of drug.

### Example 3:

The immediate release granule-2 and enteric granule-3 comprising faropenem daloxate are obtained according to the composition ratio in Table 6.

That is, faropenem daloxate, D-mannitol, croscarmellose sodium and macrogol 6000 are charged into agitating granulator and mixed. Separately, binder solution is prepared by dissolving hydroxypropylcellulose into water, and then added into agitating granulator for kneading and granulating. The resultant is then charged into basket-type granulator and granulated by extrusion method using 0.8mm screen. Subsequently, the resultant is charged into fluidized granulator for fluidized-bed drying. The dried granule is then charged into sieving machine for granulating, put trough 18 mesh sieve, and to obtain remaining particle on the 30 mesh sieve as core granule.

The seal coating solution is prepared separately by dissolving or dispersing hydroxypropyl methylcellulose 2910 and talc into water. The resultant core granule is then charged into fluidized-bed granulator, and fluidized-bed coated with spraying seal coating solution to obtain desired immediate release granule-2.

The enteric coating solution-3 is prepared separately by dissolving or dispersing hydroxypropyl methylcellulose acetate succinate, triethyl citrate, sodium lauryl sulfate and talc into water. The resultant immediate release granule-2 obtained above is then charged into fluidized-bed granulator, and fluidized-bed coated with spraying enteric coating solution-3 to obtain desired enteric granule-3.

**Table 6:**

| Formula of immediate release granule-2 and enteric granule-3 comprising faropenem daloxate | | |
|---|---|---|
| Components | Immediate release granule-2 | Enteric granule-3 |
| Faropenem daloxate(mg) (potency) | 849.0 (609.5mg potency) | 641.7 (460.7mg potency) |

| Core granule components | | |
|---|---|---|
| D-Mannitol | 19.0 | 13.8 |
| Croscarmellose sodium | 9.5 | 21.5 |
| Macrogol 6000 | 37.2 | 28.4 |
| Hydroxypropylcellulose | 28.4 | 21.5 |

| Seal coat components | | |
|---|---|---|
| Hydroxypropyl methylcellulose 2910 | 20.3 | 15.3 |
| Talc | 36.6 | 27.5 |

| Enteric coating components | | |
|---|---|---|
| Hydroxypropyl methylcellulose acetate succinate | 0.0 | 146.6 |
| Triethyl citrate | 0.0 | 39.9 |
| Sodium lauryl sulfate | 0.0 | 3.8 |
| Talc | 0.0 | 40.0 |
| Total (mg) | 1,000.0 | 1,000.0 |

The immediate release granule-2 and enteric granule-3 obtained are weighed for 90.0mg potency equivalent and 60.0mg potency equivalent quantity each, and filled in gelatin capsule along with 36.0mg of magnesium aluminometasilicate to obtain capsule-3.

**Table 7:**

| Formula of the gelatin capsule filled with immediate release granule-2 and enteric granule-3 | |
|---|---|
| Components | Capsule-3 |
| Immediate release granule-2 | 147.7 (90.0mg potency) |
| Enteric granule-3 | 130.2 (60.0mg potency) |
| Magnesium aluminometasilicate | 36.0 |
| Fill ration per capsule (mg) | 313.9 |

The capsule-3 obtained is then assessed for stability under the storage condition shown in Table 8. That is, 15 capsules of capsule-3 are sealed with 1.5g of silica gel in glass container, and stored at 40°C under 75% relative humidity for one month.

The faropenem daloxate content of these capsules after the storage are then measured by HLPC method, and dissolution of faropenem daloxate form these preparations are also conducted by means of the dissolution test according to the Second Method (paddle method) of *Japanese Pharmacopoeia.*

Both results are shown in Table 8 below.

**Table 8:**

| Stability of gelatin capsule filled with immediate release granule-2 and enteric granule-3 in glass container | | | | | |
|---|---|---|---|---|---|
| Capsule | Storage condition | Residual ratio (%) | dissolution test | | |
| | | | Water | pH 5.5 solution | pH 6.8 solution |
| | | | Release time of granule (min.) | 50% dissolution time of drug (min.) | |
| Capsule-3 | Initial | 100 | 2 | 13 | 10 |
| | 40°C/75% RH /1 month | 101.6 | 9 | 34 | 38 |
| | Content of the capsule-3 after 40°C/75% RH/1 month storage, filled into new capsule | | 2 | 13 | 8 |

As a result, although the immediate release granule-2 and enteric granule-3 filled in capsule-3 containing macrogol 6000 and triethyl citrate which is said to generate peroxides drawing in cross-linking effect of gelatin, releasing time of granule form the capsule after 40°C and 75% relative humidity conditions for 1 month storage is extended only very little compared with at the initial, owning to addition of magnesium aluminometasilicate.

Further, the time in which drug is dissolved from the granule after 40°C and 75% relative humidity conditions for 1 month storage is same as the initial for capsule-3 which content is refilled into a new and same type of gelatin capsule. More over, potency residual ratio is more than 98%.

As shown above, magnesium aluminometasilicate indicated inhibition and prevention effect even when substance, which draws in the cross-linking effect of gelatin, is comprised within the capsule.

### Example 4:

The immediate release granule-2 and enteric granule-3 obtained in Example 3 according to the composition ratio in Table 9, are weighed for 45.0mg potency equivalent and 30.0mg potency equivalent quantity each, and filled in gelatin capsule along with 20.0mg of magnesium aluminometasilicate to obtain capsule-4.

**Table 9:**

| Formula of the gelatin capsule filled with immediate release granule-2 and enteric granule-3 | |
|---|---|
| Components | Capsule-4 |
| Immediate release granule-2 | 73.9 (45.0mg potency) |
| Enteric granule-3 | 65.1 (30.0mg potency) |
| Magnesium aluminometasilicate | 20.0 |
| Fill ration per capsule (mg) | 159.0 |

The capsule-4 obtained is then assessed for stability under the storage condition shown in Table 10. That is, 10 capsules of capsule-4 are packed in press through packaging (PTP) comprised from glassine film and polyvinyl chloride sheet and these 10 press through packagings (total of 50 capsules) are heat-sealed with 4.2g of silica gel in aluminum bag and stored at 40°C under 75% relative humidity for two months. The samples after the storage are then measured for faropenem daloxate content by HPLC method, and put into dissolution test according to the Second Method (paddle method) of *Japanese Pharmacopoeia*.

Both results are shown Table 10 below.

**Table 10:**

| Stability of gelatin capsule filled with immediate release granule-2 and enteric granule-3 in press through packaging (PTP)/ aluminum bag | | | | | |
|---|---|---|---|---|---|
| Capsule | Storage condition | Residual ratio (%) | Dissolution Test | | |
| | | | Water | pH 5.5 solution | pH 6.8 solution |
| | | | Release time of granule (min.) | 50% dissolution time of drug (min.) | |
| Capsule-4 | Initial | 100 | 2 | 5 | 5 |
| | 40°C/75% RH /2 months | 99.7 | 4 | 9 | 7 |

As a result, although, the immediate release granule-2 and enteric granule-3 filled in capsule-4 containing macrogol 6000 and triethyl citrate which is said to generate peroxides drawing in cross-linking effect of gelatin, releasing time of granule form the capsule after 40°C and 75% relative humidity conditions for 2 months storage is extended only very little compared with at the initial, owning to addition of magnesium aluminometasilicate. More over, potency residual ratio is more than 98%.

As shown above, magnesium aluminometasilicate indicated inhibition and prevention effect even when packaged in PTP/aluminum bag just as in glass container in Example 3.

### Example 5:

The immediate release granule-2 and enteric granule-3 obtained in Example 3 are weighed for 90.0mg potency equivalent and 60.0mg potency equivalent quantity each according to the composition ratio in Table 11, and filled in capsule which base material is succinated gelatin along with 10.0mg of magnesium aluminometasilicate to obtain capsule-5.

**Table 11:**

| Formula of the succinated gelatin capsule filled with immediate release granule-2 and enteric granule-3 | |
|---|---|
| Components | Capsule-5 |
| Immediate release granule-2 | 147.7 (90.0mg potency) |
| Enteric granule-3 | 130.2 (60.0mg potency) |
| Magnesium aluminometasilicate | 10.0 |
| Fill ration per capsule (mg) | 287.9 |

The capsule-5 obtained is then assessed for stability under the storage condition shown in Table 12. That is, 15 capsules of capsule-5 are sealed with 1.5g of silica gel in glass container and stored at 50°C under the drifting humidity for two weeks. The samples after the storage are then measured for faropenem daloxate content by HPLC method, and put into dissolution test according to the Second Method (paddle method) of *Japanese Pharmacopoeia*.

Both results are shown Table 12 below.

**Table 12:**

| Stability of succinated gelatin capsules filled with immediate release granule-2 and enteric granule-3 in glass container | | | | | | |
|---|---|---|---|---|---|---|
| Capsule | Storage condition | Residual ratio (%) | Dissolution Test | | | |
| | | | Water | pH 5.12 soln. | pH 5.5 soln. | pH 6.8 soln. |
| | | | Release time of granule (min.) | 20% dissolution time of drug (min.) | 50% dissolution time of drug (min.) | |
| Capsule-5 | Initial | 100 | 2 | 3 | 7 | 5 |
| | 50°C /drifting humidity /2 weeks | 101.2 | 2 | 3 | 9 | 5 |

As a result, dissolution time of drug form the granule after storage is about same as the initial for the capsule comprising immediate release granule-2 and enteric granule-3. More over, potency residual ratio is more than 98%. Further, aldehyde-like substance causing cross-linking of gelatin is not generated because succinated gelatin capsule is used, and releasing time of granule from capsule is not extended.

As shown above, magnesium aluminometasilicate indicated inhibition and prevention effect even when filled into succinated gelatin capsule.

### Example 6:

The immediate release granule-2 and enteric granule-3 obtained in Example 3 are weighed for 90.0mg potency equivalent and 60.0mg potency equivalent quantity each according to the composition ratio in Table 13, and filled in capsule which base material is hydroxypropylcellulose (HPMC), i.e., cellulose derivative, along with 30.0mg of magnesium aluminometasilicate to obtain capsule-6.

**Table 13:**

| Formula of the HPMC capsule filled with immediate release granule-2 and enteric granule-3 | |
|---|---|
| Components | Capsule-6 |
| Immediate release granule-2 | 147.7 (90.0mg potency) |
| Enteric granule-3 | 130.2 (60.0mg potency) |
| Magnesium aluminometasilicate | 30.0 |
| Fill ration per capsule (mg) | 307.9 |

The capsule-6 obtained is then assessed for stability under the storage condition shown in Table 14. That is, 15 capsules of capsule-6 are sealed with 1.5g of silica gel in glass container and stored at 50°C under the drifting humidity for two weeks. The samples after the storage are then measured for faropenem daloxate content by HPLC method, and put into dissolution test according to the Second Method (paddle method) of *Japanese Pharmacopoeia.*

Both results are shown Table 14 below.

**Table 14:**

| Stability of HPMC capsules filled with immediate release granule-2 and enteric granule-3 | | | | | |
|---|---|---|---|---|---|
| Capsule | Storage condition | Residual ratio (%) | Dissolution Test | | |
| | | | Water | pH 5.5 soln. | pH 6.8 soln. |
| | | | Release time of granule (min.) | 20% dissolution time of drug (min.) | 50% dissolution time of drug (min.) |
| Capsule-6 | Initial | 100 | 2-13 | 6 | 21 |
| | 40°C/75% RH /2 months | 100.1 | 2-5 | 5 | 14 |

As a result, dissolution time of drug from the granule after storage for the immediate release granule-2 and enteric granule-3 filled in capsule-6 is about same as the initial. More over, potency residual ratio is more than 98%. Further, aldehyde-like substance causing cross-linking of gelatin is not generated because HPMC capsule was used, and releasing time of granule from capsule is not extended.

As shown above, magnesium aluminometasilicate indicated inhibition and prevention effect even when filled into HPMC capsule.

The condition of HPLC (high performance liquid chromatograph) method used for measuring faropenem daloxate content in Examples above is stated below.

Octadecylsilylize silica gel filled stainless-steel chromatographic column (Innertsil ODS-2 4.6mmF x 250mm) is used. Chromatographic column temperature is set at 40°C. For mobile-phase, solution A, which is the mixture of water comprising 0.005 v/v% of trifluoro acetic acid and acetonitrile in the ratio of 9:1, and solution B, which comprises 0.005 v/v% of trifluoro acetic acid in acetonitrile, are used. For mobile-phase control condition, the initial concentration of solution B is preset to 5%, and solution B is increased gradually to make the concentration 60% after 40 minutes from the start of the assay. The flow volume is set at 1.5mL/minute. Ultraviolet absorption detector is chosen as a detector, and 240nm measurement wavelength is used. Acetonitrile solution comprising 0.275w/v% of propyl p-hydroxybenzoate is used as internal standard solution. Residual ratio (%) after the storage is calculated against that of the initial for measured faropenem daloxate content.

In addition, the condition for dissolution test for granule and capsule in Examples above is stated below.

The dissolution test is conducted according to dissolution test method in *Japanese Pharmacopoeia.* It is conducted according to paddle method and the rotational frequency of the paddle is set at 100rpm. For the test solution, water, solution having pH 1.2 (*Japanese Pharmacopoeia,* The first solution), and Clark-Lubs buffer solution adjusted pH 5.5, pH 6.0, pH 6.5 and pH 6.8, respectively, of 900mL are used. The temperature of the test solution is set at 37±1°C. For the tested sample, 100mg potency equivalent of faropenem daloxate is charged into test solution for granule, and a capsule is contained in a sinker is charged into the solution for a capsule.

### Example 7:

The production example of a capsule using sustained release granule as long-acting preparation is stated below.

**Table 15:**

| Formula of the sustained release granule-1 comprising faropenem daloxate | | |
|---|---|---|
| Components | | Sustained release granule-1 |
| Core granule components | Faropenem daloxate | 869.8 (624.4mg potency) |
| | Ethyl acrylate /methyl methacrylate copolymer dispersion (solid content weight) | 87.0 |
| Sustained release coating components | Ethyl acrylate / methyl methacrylate copolymer dispersion (solid content weight) | 21.6 |
| | Talc | 21.6 |
| Total (mg) | | 1,000.0 |

The sustained release granule-1 is obtained according to the composition ratio stated in Table 15. That is, to the faropenem daloxate, ethyl acrylate/methyl methacrylate copolymer dispersion is added and wet kneaded. The resultant is then charged into fluidized-bed granulator for fluidized-bed drying and then put trough 30 mesh sieve to obtain core granule. The sustained release coating solution is prepared separately by dispersing ethyl acrylate/methyl methacrylate copolymer dispersion and talc into water. The resultant core granule is then charged into fluidized-bed granulator, and fluidized-bed coated with spraying sustained release coating solution to obtain the desired sustained release granule-1.

**Table 16:**

| Formula of the gelatin capsule filled with immediate release granule-2 and sustained release granule-1 | | |
|---|---|---|
| Components | Capsule-7 | Capsule-8 |
| Immediate release granule-2 | 0 | 147.7 (90.0mg potency) |
| Sustained release granule-1 | 240.2 (150.0mg potency) | 96.1 (60.0mg potency) |
| Magnesium aluminometasilicate | 30.0 | 30.0 |
| Fill ration per capsule (mg) | 270.2 | 273.8 |

The obtained sustained release granule-1 is weighed for 150.0mg potency equivalent quantity according to the composition ratio in Table 16, and filled in hard capsule along with 30.0mg of magnesium aluminometasilicate to obtain capsule-7. The immediate release granule-2 obtained in Example 3 and sustained release granule-1 are weighed for 90.0mg potency equivalent and 60.0mg potency equivalent quantity each, and filled in hard capsule along with 30.0mg of magnesium aluminometasilicate to obtain capsule-8.

### Example 8:

The immediate release granule-3 and enteric granule-4 are obtained according to the composition ratio shown in Table 17.

**Table 17:**

| Formula of immediate release granule-3 and enteric granule-4 comprising faropenem daloxate | | |
|---|---|---|
| Components | Immediate release granule-3 | Enteric granule-4 |
| Faropenem daloxate (mg) (potency) | 801.5 (575.4mg potency) | 640.5 (459.8mg potency) |

| Core granule components | | |
|---|---|---|
| D-Mannitol | 17.6 | 14.1 |
| Croscarmellose sodium | 8.8 | 21.4 |
| Macrogol 6000 | 35.7 | 28.5 |
| Hydroxypropylcellulose | 26.8 | 21.4 |

| Seal coat components | | |
|---|---|---|
| Hydroxypropyl methylcellulose 2910 | 41.0 | 16.2 |
| Magnesium aluminometasilicate | 35.2 | 0.0 |
| Talc | 33.4 | 27.3 |

| Enteric coating components | | |
|---|---|---|
| Hydroxypropyl methylcellulose acetate succinate | 0.0 | 146.8 |
| Triethyl citrate | 0.0 | 39.8 |
| Sodium lauryl sulfate | 0.0 | 4.2 |
| Talc | 0.0 | 39.8 |
| Total (mg) | 1,000.0 | 1,000.0 |

That is, faropenem daloxate, D-mannitol, croscarmellose sodium and macrogol 6000 are charged into agitating granulator and mixed. Separately, binder solution is prepared by dissolving hydroxypropylcellulose into water, and then added into agitating granulator for kneading and granulating. The resultant is then charged into basket-type granulator and granulated by extrusion method using 0.8mm screen. Subsequently, the resultant is charged into fluidized granulator for fluidized-bed drying. The dried granules are then charged into sieving machine for granulating, put trough 18 mesh sieve, and remaining particle on the 30 mesh sieve is obtained as core granule. The seal coating solution is prepared separately by dissolving or dispersing hydroxypropyl methylcellulose 2910, magnesium aluminometasilicate and talc into water. The resultant core granule obtained above is then charged into fluidized-bed granulator, and fluidized-bed coated with spraying seal coating solution to obtain desired immediate release granule-3.

The seal coating solution is prepared separately by dissolving or dispersing hydroxypropyl methylcellulose 2910, magnesium aluminometasilicate and talc into water. The core granule obtained above is charged into fluidized-bed granulator, and fluidized-bed coated with spraying above seal coat solution.

Further, hydroxypropyl methylcellulose acetate succinate, triethyl citrate and talc are dissolved or dispersed into water to prepare the enteric coating solution. The seal coated granule is charged into fluidized-bed granulator, and fluidized-bed coated with spraying the enteric coating solution to obtain desired enteric granule-4.

**Table 18:**

| Formula of the gelatin capsule filled with immediate release granule-3 and enteric granule-4 | |
|---|---|
| Components | Capsule-9 |
| Immediate release granule-3 | 156.4 (90.0mg potency) (contained 5.5mg of magnesium aluminometasilicate) |
| Enteric granule-4 | 130.5 (60.0mg potency) |
| Fill ration per capsule (mg) | 286.9 |

The immediate release granule-3 and enteric granule-4 obtained are weighed for 90.0mg potency equivalent and 60.0mg potency equivalent quantity each according to the composition ratio in Table 18, and filled in hard capsule to obtain capsule-9. The capsule-9 comprises 5.5mg of magnesium aluminometasilicate which is the constituent of seal coating of immediate release granule-3. This quantity is 2.6 weight% to the faropenem daloxate within the capsule.

The capsule-9 obtained is then assessed for stability under the maintenance condition shown in Table 19. That is, 12 capsules of capsule-9 are sealed with 1.0g of silica gel in glass container and stored at 40°C under 75% relative humidity for two months. The samples after the storage are then measure for faropenem daloxate content by HPLC method, and put into dissolution test according to the Second Method (paddle method) of *Japanese Pharmacopoeia.*

Both results are shown in Table below.

**Table 19:**

| Stability of gelatin capsules filled with immediate release granule-3 and enteric granule-4 | | | | |
|---|---|---|---|---|
| Capsule | Storage condition | Dissolution test | | |
| | | Water | pH 5.5 solution | pH 6.8 solution |
| | | Release time of granule (min.) | 50% dissolution time of drug (min.) | |
| Capsule-9 | Initial | 2 | 9 | 10 |
| | 40°C/75% RH /2 months | More than 30 (2*) | More than 80 (10*) | More than 60 (9*) |
| | | *: Content of the capsule-9 after stored at 40°C under 75% RH for 2 months, is filled into same type of new gelatin capsule for dissolution test | | |

As a result, dissolution time of drug form the granule after storing at 40°C under 75% relative humidity for 2 months is extended compared to the initial due to the fact that composition quantity of the magnesium aluminometasilicate is as little as 5.5mg. However, after the content of the capsule-9 is stored at 40°C under 75% RH for 2 months and filled into same type of new gelatin capsule for dissolution test, dissolution time of drug from the granule is same as the initial.

As shown above, magnesium aluminometasilicate indicated inhibition and prevention effect even when it is comprised in very small quantity.

### Example 9:

The immediate release granule-3 and enteric granule-4 obtained in Example 8 are weighed for 150.0mg potency equivalent quantity each according to the composition ratio in Table 20, and filled in hard gelatin capsule to obtain capsule-10 and capsule-11.

**Table 20:**

| Formula of the gelatin capsule filled with immediate release granule-3 and enteric granule-4 | | |
|---|---|---|
| Components | Capsule-10 | Capsule-11 |
| Immediate release granule-3 | 260.7 (150.0mg potency) | 0.0 |
| Enteric granule-4 | 0.0 | 326.2 (150.0mg potency) |
| Fill ration per capsule (mg) | 260.7 | 326.2 |

The obtained capsule-10 and capsule-11 are oral administered after eating to humans (5 adults), and their blood samples are taken with time up to 12 hours after the administration. Then the faropenem concentration as an antibacterial activator of faropenem daloxate in blood plasma and pharmacokinetic parameter are calculated.

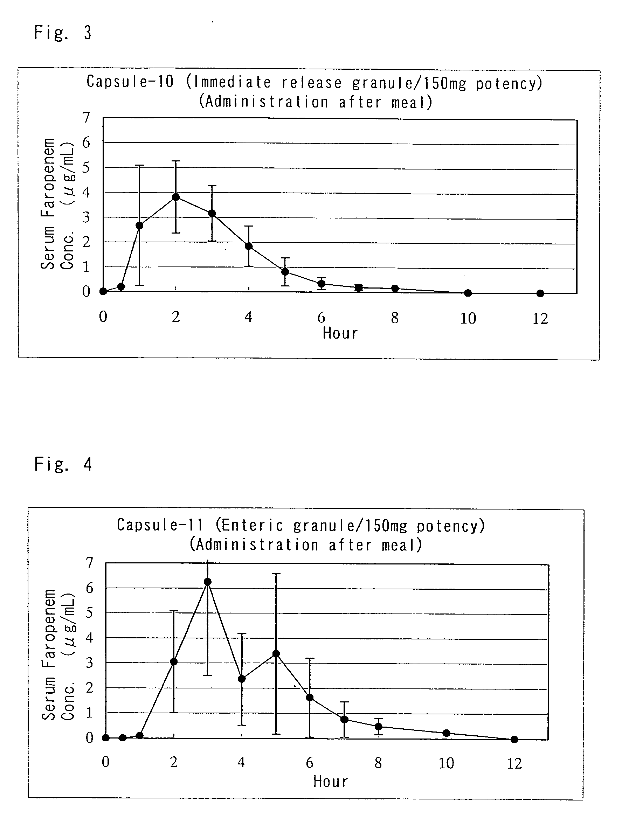

Faropenem concentration in blood plasma is shown in Figure 3 and Figure 4 and pharmacokinetic parameters are shown in Table 21.

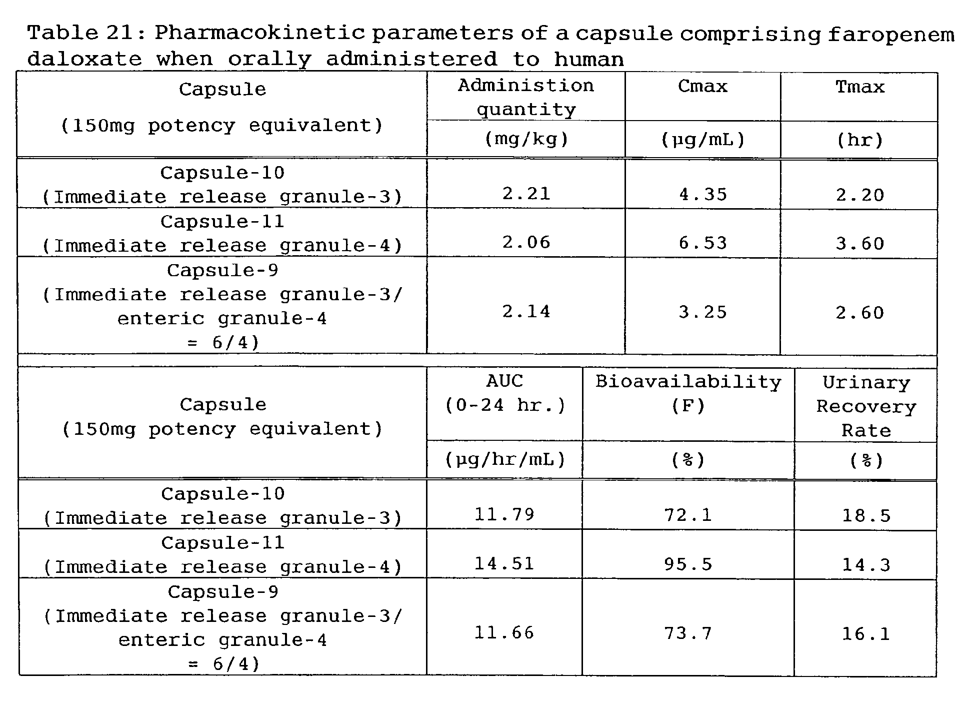

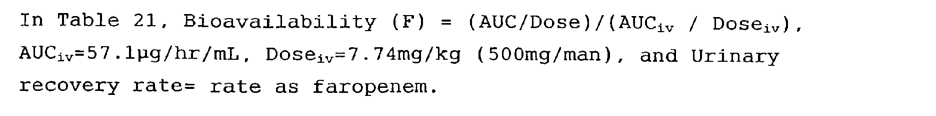

As a result, when capsule-10 filled with immediate release granule-3 and capsule-11 filled with enteric granule-4 are orally administered, maximum plasma concentration arrival time (Tmax) is 2.2 hours and 3.6 hours respectively. Maximum plasma concentration (Cmax) andbioavailability of use for capsule-10 is 4.35µg/mL and 72.1%, and for capsule-11 is 6.53µg/mL and 95.5%. In another words, they are both higher in capsule-11. From the result, to keep the effective faropenem plasma concentration as long as possible, immediate release granule-3 with lower bioavailability of use may be comprised little more than enteric granule-4. Accordingly, composition granule in which composition ratio of immediate release granule-3 to enteric granule-4 to be 6:4 is chosen.

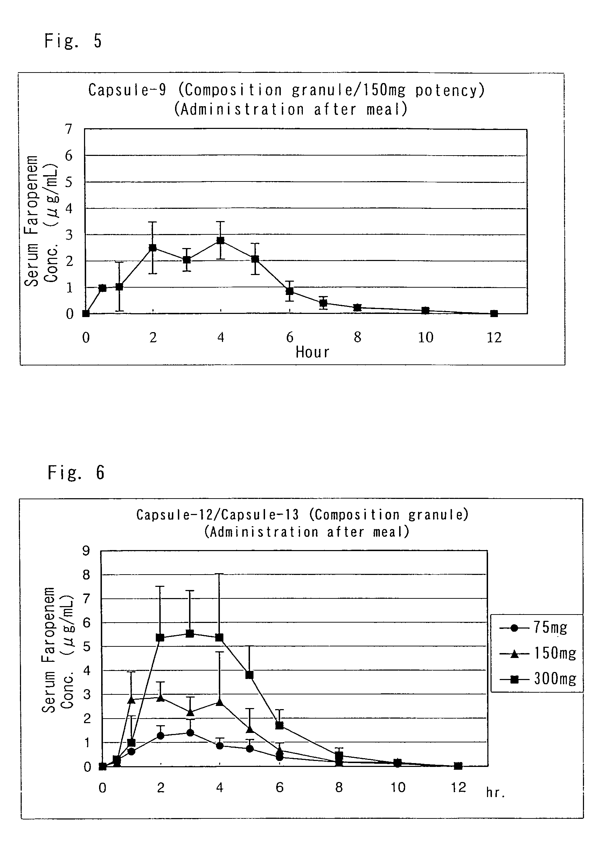

In fact, capsule-9 obtained in Example 8 which composition ratio of immediate release granule-3 to enteric granule-4 is 6:4 is orally administered to human (5 adults) after eating, and their blood samples are taken with time up to 12 hours after the administration. Then the faropenem concentration as a antibacterial activator of faropenem daloxate in blood plasma and pharmacokinetic parameters are calculated. Transition of faropenem concentration in blood plasma is shown in Figure 5 and pharmacokinetic parameters are shown in Table 21.

As a result, plasma faropenem concentration has reached 1µg/mL after 30 minutes of oral administration (see Figure 5). On the other hand, plasma faropenem concentration has not reached 1µg/mL even after an hour of oral administration for capsule-11. It is confirmed from the result that plasma faropenem concentration is raised promptly after oral administration due to composition granule is made in composition ratio of immediate release granule as 6 to enteric granule as 4. Additionally, more than 1µg/mL of faropenem concentration can be kept until 6 hours after the administration.

### Example 10:

The immediate release granule-2 and enteric granule-3 obtained in Example 3 are weighed for 45.0mg and 30.0mg potency equivalent quantity each according to the composition ratio in Table 22, and filled in hard gelatin capsule with 10.0mg of magnesium aluminometasilicate to obtain capsule-12. Separately, the immediate release granule-2 and enteric granule-3 are weighed for 90.0mg and 60.0mg potency equivalent quantity each according to the composition ratio in Table 22, and filled in hard gelatin capsule with 20.0mg of magnesium aluminometasilicate to obtain capsule-13.

**Table 22:**

| Formula of the gelatin capsule filled with immediate release granule-2 and enteric granule-3 | | |
|---|---|---|
| Components | Capsule-12 | Capsule-13 |
| Immediate release granule-2 | 73.9 (45.0mg potency) | 147.7 (90.0mg potency) |
| Enteric granule-3 | 65.1 (30.0mg potency) | 130.2 (60.0mg potency) |
| Magnesium aluminometasilicate | 10.0 | 20.0 |
| Fill ration per capsule (mg) | 149.0 | 297.9 |

One capsule (75mg potency equivalent) of the obtained capsule-12 , one capsule (150mg potency equivalent) or two capsules (300mg potency equivalent) of capsule-13 are oral administered after eating to humans (6 adults), and their blood samples are taken with time up to 12 hours after the administration. Then the faropenem concentration as an antibacterial activator of faropenem daloxate in blood plasma and pharmacokinetic parameters are calculated.

Faropenem concentration in blood plasma is shown in Figure 6 and pharmacokinetic parameters are shown in Table 23.

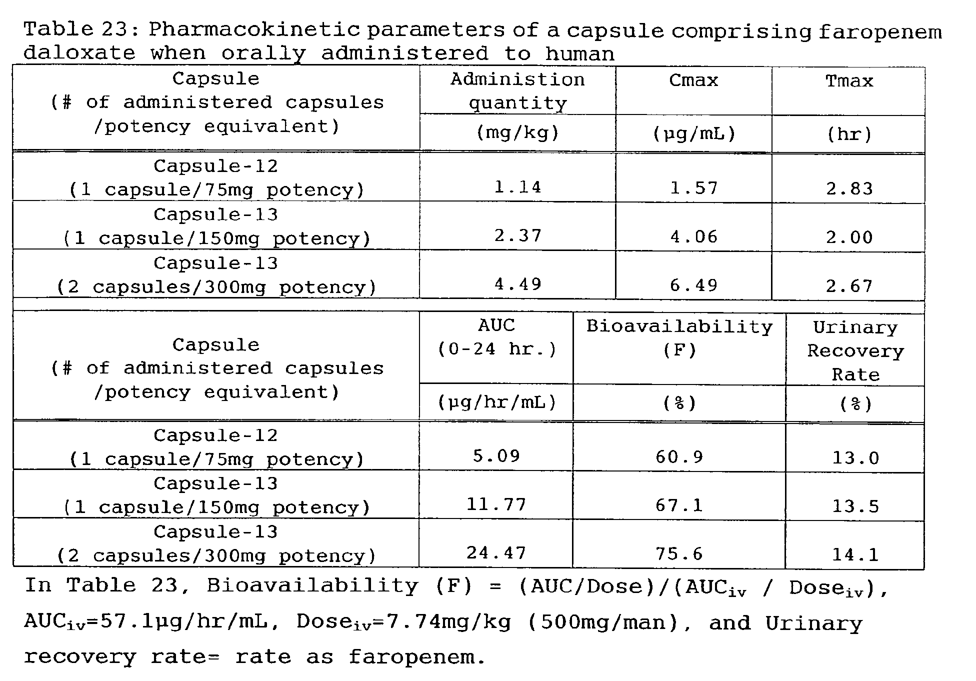

As a result, when one capsule (75mg potency equivalent) of capsule-12 and one capsule (150mg potency equivalent) or two capsules (300mg potency equivalent) of capsule-13 are orally administered, maximum plasma concentration arrival time (Tmax) is 2.83 hours, 2.00 hours and 2.67 hours respectively, and they are almost the same. The AUC [area under the (blood concentration-time) curve] is 5.09µg/hr/mL for capsule-12 (75mg potency equivalent), 11.77µg/hr/mL for capsule-13 (150mg potency equivalent) and 24.47µg/hr/mL for capsule-13 (300mg potency equivalent) respectively. It can be said that AUC increased proportionately to administration dosage.

The bioavailability and the urine recovery rate are 60.9% and 13.0% for capsule-12 (75mg potency equivalent), 67.1% and 13.5% for capsule-13 (150mg potency equivalent) and 75.6% and 14.1% for capsule-13 (300mg potency equivalent) respectively, and dose not vary depending on the administration dosage. Accordingly, when a capsule comprising composition granule in which composition ratio of immediate release granule-2 to enteric granule-3 to be 6:4 and magnesium aluminometasilicate is administered within the range of faropenem daloxitate being 75mg to 300mg potency equivalent, it is assumed to be liner pharmacokinetic, and thus easy to plan administration.

For above Example, blood and urine process method and high performance liquid chromatograph method for plasma and urine faropenem content measurement are stated below.

The collected blood and urine are pretreated before using. That is, supernatant is dispensed after blood and urine are centrifuged for 15 minutes at 3, 000 rpm. For blood, 200µL of acetonitrile is added to 200µL of supernatant (plasma) and stirred. The resultant is then centrifuged for 15 minutes at 4°C and 12,000rpm. The supernatant 200µL is diluted by 800µL of 10mM phosphoric buffer, and of 200µL is charged into high performance liquid chromatograph. For urine, 200µL of acetonitrile is added to 200µL of supernatant (plasma) and stirred. The 100µL of supernatant is added to 900µL of 10mM phosphoric buffer and stirred, and the 50µL of resultant is then diluted by 900µL of 10mM phosphoric buffer. Of 200µL of sample is charged into high performance liquid chromatograph (HPLC).

The condition for HPLC (high performance liquid chromatograph) is as below. That is, octadecylsilylize silica gel filled stainless-steel chromatographic column (Innertsil ODS-2 4.6mmF x 250mm) is used. Chromatographic column temperature is set at room temperature. For mobile-phase, a solution which is the mixture of 680mL of 20mM sodium dihydrogen phosphate solution adjusted to pH2 by phosphoric acid and 320mL of acetonitrile are used. The flow volume is set at 1mL/minute. Ultraviolet absorption detector is chosen as a detector, and 318nm measurement wavelength is used.

### Industrial applicability

As stated above, the present invention provides a hard capsule stably comprising any drug filled within, and more specifically, a hard capsule in which insolubilization of the hard gelatin capsule and degeneration of the functional coating applied to drug due to the gaseous substance generated by degradation of drugs are prevented by adding inorganic substance.

It also makes the preparation design to carry out the desired function well as in long lasting agent possible because degeneration of functional coating dose not occur, thus enabling to hold the target function.

Additionally, the present invention provides a capsule containing faropenem daloxate which is a penem antibiotic with excellent antibacterial activity and possessing well sustainability of antibacterial effect in oral administration. Thus making a great contribution for medical progress.

## Claims

1. A hard capsule in which an inorganic substance is contained with a drug filled therein.

2. A hard capsule according to claim 1, wherein base material of the hard capsule is gelatin.

3. A hard capsule according to claim 1 or 2, wherein the inorganic substance contained therein is any one or more than one selected from the group consisting hydrated aluminum silicate, synthetic aluminum silicate, light anhydrous silicic acid, hydrated silicon dioxide and magnesium aluminometasilicate.

4. A hard capsule according to claim 1 or 2, wherein the inorganic substance is magnesium aluminometasilicate.

5. A hard capsule according to any one of claims 1 to 4, wherein the quantity of inorganic substance is within the range of 0.1 to 100 weight % of the drug filled within.

6. A hard capsule according to any one of claims 1 to 5, wherein the drug filled in the hard capsule is in solid or semisolid form.

7. A hard capsule according to claim 6, wherein solid and semisolid drug is fine granule or granule.

8. A hard capsule according to any one of claims 1 to 7, wherein the drug filled within the hard capsule is applied with functional coating.

9. A hard capsule according to claim 7, wherein the functional coating to be applied to the drug is an enteric coating.

10. A hard capsule according to claim 7, wherein the functional coating to be applied to the drug is a gastric coating.

11. A hard capsule according to any one of claims 1 to 10, wherein the drug filled within is a composition of a drug applied with functional coating and a drug without functional coating.

12. A hard capsule according to claim 11, wherein the ratio of the composition of a drug applied with functional coating and a drug without functional coating is within the range of 3:7 to 7:3 in weight.

13. A hard capsule according to any one of claims 1 to 7, wherein the drug filled within the hard capsule is a composition of a drug applied with enteric coating and a drug applied with gastric coating.

14. A hard capsule according to claim 13, wherein the ratio of the composition of the drug applied with enteric coating to the drug applied with gastric coating is within the range of 3:7 to 7:3 in weight.

15. A hard capsule according to any one of claims 1 to 14, wherein the drug filled in the hard capsule is penem antibiotic.

16. A hard capsule according to claim 15, wherein the penem antibiotic filled in the hard capsule is (+)-(5R,6S)-6-[(R)-1-hydroxyethyl]-7-oxo-3-[(R)-2-tetrahydrofuryl]-4-thia-1-azabicyclo[3.2.0]hept-2-ene-2-carboxylic acid or a pharmaceutically acceptable salt thereof and a derivative thereof, or pharmacologically acceptable salt of derivative thereof.

17. A hard capsule according to claim 15, wherein a penem antibiotic filled in the hard capsule is (5-methyl-2-oxo-1,3-dioxolene-4-yl)methyl (5R,6S)-6-[(R)-1-hydroxyethyl]-7-oxo-3-[(R)-2-tetrahydrofuryl]-4-thia-1-azabicyclo[3.2.0]hept-2-ene-2-carboxylate.

18. A hard capsule according to any one of claims 15 to 17, wherein the quantity of inorganic substance is within the range of 1 to 100 by weight% of the penem antibiotic filled within.

19. A hard capsule according to any one of claims 15 to 17, wherein the quantity of inorganic substance is within the range of 1 to 25 by weight% of penem antibiotic filled within.

20. A hard capsule according to any one of claims 15 to 19, wherein penem antibiotic filled within is a mixture of penem antibiotic applied with functional coating and penem antibiotic without functional coating.

21. A hard capsule according to claim 20, wherein the ratio of a mixture of penem antibiotic applied with functional coating to penem antibiotic without functional coating is within the range of 5:5 to 7:3 in weight.

22. A hard capsule according to any one of claims 15 to 19, wherein penem antibiotic filled within the hard capsule is a mixture of penem antibiotic applied with enteric coating and penem antibiotic applied with gastric coating.

23. A hard capsule according to claim 22, wherein the ratio of a mixture of penem antibiotic applied with enteric coating to penem antibiotic applied with gastric coating is within the range of 5:5 to 7:3 in weight.
